# EUROPÄISCHE PATENTSCHRIFT

(11) **EP 2 705 859 B1**
(45) Veröffentlichungstag und Bekanntmachung des Hinweises auf die Patenterteilung: **07.10.2015**
(21) Anmeldenummer: 13000513.5
(22) Anmeldetag: 01.02.2013
(51) Int. Cl.: A61L 27/18, A61L 27/34

(54) **Beschichtete Intraokularlinse und ihre Herstellung**
Coated intraocular lens and its production
Lentille intraoculaire revêtue et sa fabrication

(43) Veröffentlichungstag der Anmeldung: 12.03.2014
(73) Patentinhaber: LensWista AG, 12489 Berlin (DE)
(72) Erfinder: Görne, Martin, 22337 Hamburg (DE); Kordick, Thomas, 63773 Goldbach (DE)
(74) Vertreter: Diehl & Partner GbR

(56) Entgegenhaltungen:
- EP-A1- 0 834 352
- EP-A2- 0 908 476
- US-A- 5 344 449
- US-A1- 2004 166 232
- US-B1- 6 436 481

## Beschreibung

Die Erfindung betrifft eine beschichtete Intraokularlinse, IOL, und ein Verfahren zu ihrer Herstellung.

Derartige Linsen werden insbesondere nach Eintrübung der natürlichen Augenlinse als Ersatz der natürlichen Augenlinse im Rahmen einer Kataraktoperation implantiert. Bekannte Linsenkörper bestehen aus hydrophobem Material, insbesondere Copolymeren, welche Acrylat oder Methacrylat enthalten. Zur Herabsetzung der Klebrigkeit ist es auch bekannt, dem Linsenmaterial fluoriertes Acrylat oder Methacrylat zuzusetzen (WO 2007/062864). Ferner ist es bekannt, für die Intraokularlinse ein Linsenmaterial zu verwenden, welches einen hohen Brechungsindex aufweist um eine geringe Linsendicke zu ermöglichen. Derartige, falt- oder rollbare Linsen können durch relativ kleine Schnitte mit Hilfe von Injektoren, wie sie aus US 6 355 046 B2 bekannt sind, in das Auge implantiert werden.

Aufgrund des Brechungsindex-Unterschiedes des Linsenmaterials gegenüber dem im Auge umgebenden Medium, nämlich dem Kammerwasser in der Vorderkammer des Auges und Glaskörper an der Linsenrückseite, kommt es an den Grenzflächen zu Lichtreflexion. Diese ist umso ausgeprägter je höher der Brechungsindex-Unterschied zwischen dem Linsenmaterial und dem umgebenden Medium ist. Außerdem werden bei der Herstellung der Intraokularlinsen die Oberflächen mittels Submikrometer-Technologie spanabhebend bearbeitet um einen anschließenden Polierschritt zu vermeiden. So behandelte Linsenoberflächen weisen jedoch noch Mikrorauigkeiten auf, die zu Lichtstreuung beitragen können.

Eine Aufgabe der Erfindung ist es, eine Intraokularlinse zu schaffen, bei welcher ein möglichst kontinuierlicher lichtoptischer Übergang zwischen dem die im Auge implantierte Linse umgebenden Medium und der Linsenoberfläche stattfindet.

Die vorliegende Erfindung betrifft auch die Oberflächenbehandlung von Werkstücken auf Basis von Biomaterialien und bezieht sich im Besonderen auf ein dauerhaftes Hydrophilieren von Oberflächen solcher Werkstücke, insbesondere IOLs mittels plasmaunterstützter chemischer Gasphasenabscheidung (PECVD) und nachfolgender chemischer Gasphasenabscheidung (CVD).

An die biologische Verträglichkeit von Werkstücken, die zum vorübergehenden oder dauerhaften Einsatz an Organen von Menschen oder Tieren vorgesehen sind, wie beispielsweise Intraokularlinsen, werden, um Entzündungsprozesse zu vermeiden, hohe Anforderungen gestellt. Um eine hohe Biokompatibilität sicherzustellen, werden zur Herstellung solcher Werkstücke Materialien verwendet, deren Eigenschaften sie sowohl für den jeweiligen Einsatzzweck als auch den damit verbundenen Gewebekontakt prädestinieren. Die auch als Biokompatibilität bezeichnete biologische Verträglichkeit von Materialien wird zu einem wesentlichen Teil durch deren Oberflächeneigenschaften beeinflusst. Bei IOLs ist eine hydrophile Oberfläche entscheidend für eine gute Biokompatibilität.

Ein biokompatibles Hydrophilieren von Oberflächen polymerer Biomaterialien kann, wie z. B. in der internationalen Anmeldung WO 99/57177 beschrieben ist, durch ein Modifizieren der Polymeroberfläche mittels Plasmaoxidation erzielt werden. Es hat sich jedoch gezeigt, dass solcherart hydrophilierte Oberflächen nicht in ausreichendem Maß langzeitstabil sind.

Eine dauerhaftere Hydrophilierung polymerer Biomaterialoberflächen wird durch Beschichten dieser mit einem hydrophilen, biokompatiblen Werkstoff erreicht. Zum Herstellen hydrophiler Oberflächen an Kontaktlinsen aus Polymethylmethacrylat (PMMA) wird in der Patentschrift US 5,080,924 z. B. ein Plasmaabscheideprozess zum Aufpolymerisieren der Oberflächen mit Polyacrylsäure vorgeschlagen. Die aufpolymerisierten PAA-Oberflächen zeigen Benetzungswinkel für Wasser aus dem Bereich von 35 bis 50 Grad und sind für eine ausreichende Benetzung der Materialoberflächen zu groß. Zur weiteren Verringerung des Benetzungswinkels muss die Beschichtung weiterbehandelt werden, beispielsweise durch Aufbringen eines von Acrylsäure verschiedenen weiteren biokompatiblen Materials, das sich mit der Polyacrylsäure vernetzt. Ein solcher mehrere Materialschichten umfassender Prozess erfordert jedoch einen höheren apparativen Aufwand und resultiert auch in längeren Beschichtungszeiten.

Das Dokument US 6,436,481 B1 offenbart ein Verfahren zum Herstellen von beschichteten Gegenständen mittels *after-glow-*Plasma-induzierter Polymerisation ungesättigter Verbindungen mit gewissen reaktiven funktionellen Gruppen. In einem ersten Schritt wird im Argonplasma aktiviert, dann ohne Plasmaeinwirkung mit der Verbindung beschichtet, dann mit Plasmaeinwirkung mit der Verbindung beschichtet und dann wieder ohne Plasmaeinwirkung mit der Verbindung beschichtet.

Das Dokument EP 0 834 352 A1 offenbart ebenfalls *after-glow-*Plasma-induzierte Beschichtungen, die dadurch erhalten werden, dass in einem ersten Schritt im Argonplasma aktiviert, dann ohne Plasmaeinwirkung beschichtet, und abschließend mit Plasmaeinwirkung beschichtet wird.

Das Dokument EP 0 908 476 A2 offenbart eine beschichteten Formartikel mit einem Benetzungswinkel von unter 60°.

Ausgehend vom Dargelegten ist es daher wünschenswert, ein weniger komplexes biokompatibles Beschichten polymerer Biomaterialien anzugeben, insbesondere von IOLs, das eine langzeitstabile Oberflächenhydrophilierung mit Wasserbenetzungswinkeln von 15 Grad und darunter ermöglicht.

Die vorliegende Erfindung schlägt dazu die Intraokularlinse gemäß Anspruch 1 vor.

Eine derartige Linse besitzt einen hydrophoben Linsenkörper an dessen Oberfläche eine hydrophile Schicht vorgesehen ist. Bevorzugt besteht der Linsenkörper aus einem hydrophoben und falt- oder rollbaren Polymermaterial wie Silikonkautschuk. Die hydrophile Schicht besteht aus hydrophilem Acrylat mit guter Gewebe- und Blutverträglichkeit. Diese Beschichtung verhindert das Anheften von Fibrin und adhärenten Zellen und wirken dadurch einer postoperativen Membranbildung (Sekundär-Katarakt) entgegen.

Bei dem hydrophoben Linsenmaterial handelt es sich zweckmäßig um ein solches, welches weniger als 5 Vol% Wasser aufnimmt. Vorzugsweise handelt es sich um falt- oder rollbare Linsenkörper.

Die vorliegende Erfindung schlägt ferner das Verfahren gemäß Anspruch 10 vor.

Die aus Silikonkautschuk bestehenden Linsenkörper können im Molding-Verfahren hergestellt werden. Dabei überträgt sich die Rauigkeit der Mold-Oberflächen auf die Linsenoberfläche. Durch Aufbringen der hydrophilen Beschichtung auf diese Oberfläche werden die Rauigkeiten geglättet und Lichtstreuung weitestgehend vermieden.

Der Brechungsindex der hydrophilen Beschichtung ist zweckmäßig so gewählt, dass er zwischen dem Brechungsindex des Linsenmaterials und dem Brechungsindex des umgebenden Mediums im Auge, welches im Wesentlichen das Kammerwasser und der Glaskörper sind, liegt. Das bedeutet, dass der Brechungsindex vorzugsweise zwischen n=1,336 (Kammerwasser) bzw. 1,38 (Glaskörper) und n=1,56 (aus WO 2007/062864 A2 bekanntes Polymermaterial) vorzugsweise gewählt werden kann. Da der Brechungsindex der hydrophilen Acrylatschicht zwischen dem des hydrophoben Linsenmaterials, aus dem der Linsenkörper besteht, und dem des umgebenden Mediums im Auge, nämlich des Kammerwassers und des Glaskörpers liegt, erreicht man einen angenähert kontinuierlichen lichtoptischen Übergang zwischen dem Kammerwasser, dem Linsenkörper und dem Glaskörper, wodurch Lichtreflexion und durch die verbleibenden Mikrorauigkeiten verursachte Lichtstreuung reduziert werden oder sich vermeiden lassen.

Durch die hydrophile Beschichtung wird außerdem die Gleiteigenschaft der Linse beim Implantieren mit Hilfe eines Injektors oder anderem Implantierwerkzeug, z. B. Pinzette verbessert. Derartige Injektoren sind beispielsweise aus US 6 355 046 B2 bekannt und dienen zum Halten oder Rollen der zu implantierenden Linse. Beim Implantieren wird die gefaltete oder gerollte Linse durch eine Kanüle, welche durch eine minimale Öffnung im Auge gesteckt ist, in das Auge implantiert.

Da die Innenwandung der Kanüle und der Faltkartusche, welche bei den bekannten Injektoren vorgesehen sind, in der Regel aus Polyethylen oder Polypropylen bestehen ist die Gleiteigenschaft an der Innenwandung der Kartusche und der Kanüle relativ gering, sodass ohne zusätzliche Gleitmittel der Injiziervorgang erschwert ist. Die zur Anwendung kommenden Gleitmittel können dabei in das Auge gelangen.

Gewünschtenfalls kann die Intraokularlinse, IOL, in einem Injektor aufbewahrt werden, wobei der Injektor ein Einmalteil darstellt. Aufgrund der hydrophilen Beschichtung an der Intraokularlinse und ggf. an der Innenwand der Injektionskanüle und ggf. an der Faltkartusche des Injektors erreicht man eine Verbesserung der Gleiteigenschaften und somit eine Erleichterung beim Implantieren der Intraokularlinse.

Das Beschichten umfasst ein Verfahren zum Hydrophilieren der Oberflächen von Intraokularlinsen, wobei das Verfahren einen Schritt (a) umfasst zum Reinigen und Aktivieren der Linsenoberflächen im Rahmen einer Vorbehandlung mit einem auf Grundlage eines Inertgases gebildeten Hochfrequenzgasplasmas, einen Schritt (b) zum Vorbeschichten der vorbehandelten Linsenoberflächen mit Polyacrylsäure unter Verwendung eines aus einer Gasmischung erzeugten Hochfrequenzgasplasmas, wobei sich die Gasmischung aus einem Inertgas und einem aus biokompatiblen, polymerisierbaren carboxygruppenhaltigen Monomeren gebildeten ersten Gas zusammensetzt, und einen Schritt (c) zum Folgebeschichten der vorbeschichteten Linsenoberflächen unter Verwendung eines im Wesentlichen Acrylsäuremonomere enthaltenden zweiten Gases.

Das Beschichten umfasst ferner das Bereitstellen einer IOL mit einer hydrophilierenden Oberflächenbeschichtung aus Polyacrylsäure, die nach einem die vorgehend spezifizierten Schritte umfassenden Verfahren erhältlich ist, wobei der Benetzungswinkel von Wasser an der mit Polyacrylsäure beschichteten Linsenoberfläche einen Wert aus dem Bereich von 2 bis weniger als 10 Grad oder aus dem Bereich unter 2 Grad aufweist.

Die mit dem spezifizierten Verfahren beschichteten IOLs weisen eine dauerhaft hydrophile Oberfläche ausgezeichneter Benetzungsfähigkeit auf, die bei Kontakt mit Körpergewebe in einer guten Bioverträglichkeit resultiert, wodurch Reizungen des Auges bei entsprechend beschichteten IOLs weniger häufig auftreten.

Bei vorteilhaften Ausführungsformen des Verfahren sind die das erste Gas bildenden biokompatiblen, polymerisierbaren carboxygruppenhaltigen Monomere ausgewählt sind unter (Meth)Acrylsäure und (Meth)Acrylsäureanhydrid, wodurch im Hochfrequenzplasma ein hoher Anteil von Acrylsäuremonomeren erzeugt wird, die sich an der in Schritt (a) des Verfahrens aktivierten Linsenoberfläche unter Ausbildung kovalenter Bindungen anlagern.

Bei weiter vorteilhaften Ausführungsformen enthält das in Schritt (a) des Verfahrens zur Ausbildung des Hochfrequenzplasmas verwendete Gas das erste Gas in einer Menge, die einem Partialdruck von weniger als einem Zehntel des Partialdrucks des Inertgases entspricht, so dass eine effektive Reinigung und Aktivierung der Linsenoberfläche sichergestellt ist.

Um eine stabile Anlagerung von Acrylsäuremonomeren an die Linsenoberfläche zu erhalten wird bei Ausführungsformen in Schritt (b) eine Gasmischung verwendet, bei der der Partialdruck des ersten Gases mindestens ein Viertel und maximal das Doppelte des Partialdrucks des Inertgases beträgt.

Im Hinblick auf den Erhalt einer dichten und stabilen Polyacrylsäurebeschichtung beträgt der Partialdruck des Inertgases am in Schritt (c) verwendeten zweiten Gas bei Ausführungsformen vorzugsweise weniger als ein Zehntel des Partialdrucks des von Acrylsäuremonomeren gebildeten Gases.

In Ausführungsformen wird Argon als Inertgas verwendet.

Zur effektiven Kontrolle des Vorbeschichtungsprozesses wird bei Ausführungsformen die in Schritt (b) aufgebrachte Beschichtung mithilfe einer Schichtdickenkontrolleinrichtung überwacht und bei Erreichen eines aus dem Bereich von 50 bis 400 Å ausgewählten Schichtdickenwerts beendet.

Bei besonders bevorzugten Ausführungsformen, bei denen Benetzungswinkel aus dem Bereich von 2 bis weniger als 10 Grad erreicht werden, weist der Druck des Inertgases für das Hochfrequenzplasma in Schritt (a) einen Wert aus dem Bereich von 15 bis 60 mTorr (ca. 2 bis 8 Pa) und der Druck des ersten Gases für das Hochfrequenzplasma in Schritt (b) einen Wert aus dem Bereich von 30 bis 90 mTorr (ca. 4 bis 12 Pa) auf.

Zur Fixierung der Acrylsäurepolymerbeschichtung auf den Linsenoberflächen umfassen Ausführungsformen ferner einen Schritt (cb), der unmittelbar an Schritt (b) anschließend ein Drosseln der Inertgaszufuhr und ein Zuführen des zweiten Gases umfasst, wobei der Druck des zweiten Gases in Schritt (c) weniger als 0,3 mTorr (ca. 40 mPa) beträgt.

Zur Begünstigung einer Anlagerung und Vernetzung von Acrylsäuremonomeren an der vorbeschichteten Linsenoberfläche weisen Ausführungsformen ferner einen Schritt (bc) auf, der unmittelbar an Schritt (b) oder, wenn ausgeführt, an Schritt (cb) anschließend ein Ausschalten des Hochfrequenzplasmas, ein Unterbrechen der Inertgaszufuhr und ein Zuführen des zweiten Gases umfasst, wobei der Druck des zweiten Gases in Schritt (c) zwischen 1,5 und 6 Torr (ca. 0,13 bis 0,8 kPa) beträgt.

Zur Verbesserung der Bioverträglichkeit weisen Ausführungsformen ferner einen an Schritt (c) anschließenden weiteren Schritt (d) zum Entfernen wasserlöslicher Bestandteile aus der Hydrophilierungsschicht mittels Spülen der beschichteten IOL in einem hydrophilen Lösungsmittel wie z. B. in einer isotonischen Kochsalzlösung oder in demineralisiertem Wasser auf, optional gefolgt von Vakuumtrocknen.

Bei weiterhin bevorzugten Ausführungsformen weist die IOL zumindest an ihrer Oberfläche ein Material auf, das überwiegend oder im Wesentlichen aus einem Silicon, insbesondere Poly(dimethylsiloxan), einem Siliconhydrogel gebildet ist.

Bei Ausführungsformen handelt es sich bei den Linsen um Silikon-IOLs. Die mit dem Verfahren hergestellte hydrophilierende Oberflächenbeschichtung dieser Linsen wird von einer PAA-Schicht mit einer durchschnittlichen Dicke von 5 bis 40 µm gebildet.

Weitere Merkmale der Erfindung ergeben sich aus der nachfolgenden Beschreibung von Ausführungsbeispielen in Verbindung mit den Ansprüchen sowie den Figuren. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung von Ausführungsbeispielen wird auf die beiliegenden Figuren Bezug genommen, von denen
- Figur 1: schematisch eine schnittbildliche Darstellung einer Intraokularlinse als Ausführungsbeispiel der Erfindung zeigt;
- Figur 2: eine schematische Darstellung zur Veranschaulichung eines Systems für eine biokompatible Beschichtung von polymeren Biomaterialien zeigt,
- Figur 3: ein Flussdiagramm zur Veranschaulichung der für eine Beschichtung polymerer Biomaterialien mit Polyacrylsäure wesentlichen Verfahrensschritte zeigt, und
- Figur 4: ein Fluoreszenz-Diagramm zur Veranschaulichung der mit dem Verfahren nach Figur 3 erreichten Schichtdicke zeigt.

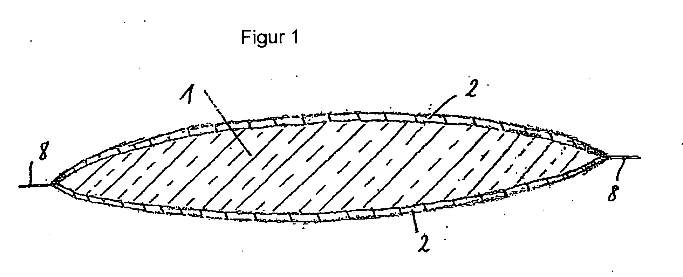

Das in der Figur 1 dargestellte Ausführungsbeispiel einer Intraokularlinse besitzt einen Linsenkörper 1 aus hydrophobem Silikon. Vorzugsweise ist das Linsenmaterial so ausgebildet, dass der Linsenkörper 1 gefaltet oder gerollt werden kann. Die Oberfläche des Linsenkörpers 1 besitzt eine Mikrorauigkeit, welche aus dem Molding herrührt. Auf die mikroraue Oberfläche des Linsenkörpers 1 ist eine hydrophile Schicht 2 aus einem hydrophilen Acrylat aufgebracht. Die Beschichtung erfolgt mit Hilfe des nachstehend erläuterten Beschichtungsverfahrens. Am Rand des optischen Linsenkörpers kann eine Haptik 8 in Form von Fäden oder Stützstegen oder in Form einer den Linsenkörper 1 ganz oder teilweise umfassenden Stützumrandung vorgesehen sein.

Auf den Linsenkörper 1 aus einem hydrophoben Material, beispielsweise Silikon, wird nach Aktivierung der Oberfläche des Linsenkörpers 1 die hydrophile Schicht 2 aufgebracht. Die Aktivierung der Oberfläche erfolgt durch Plasmaaktivierung, z. B. unter Verwendung eines Stickstoff- oder Argon-Plasmas. Auf die aktivierte Oberfläche wird das Monomer des hydrophilen Acrylats aufgebracht. Anschließend wird die Oberfläche gewaschen und bei etwa 35°C vakuumgetrocknet. Die Haptik 8 braucht nicht aus Silikon zu bestehen, sondern kann z. B. aus PMMA, PP oder Polyimid bestehen.

Hierdurch entsteht in einem Oberflächenbereich des hydrophoben Linsenmaterials ein interpenetrierendes Polymer-Netzwerk. Dabei werden Moleküle des hydrophilen Acrylats in die Oberfläche des hydrophoben Linsenmaterials eingelagert und nach Polymerisation mit diesem vernetzt. Durch die oben beschriebene Aktivierung der Oberfläche des Linsenkörpers wird dieser Vorgang noch unterstützt. Hierdurch entsteht an der Oberfläche des Linsenkörpers mit dem interpenetrierenden Netzwerk zwischen dem Kern des Linsenkörpers und der hydrophilen Schicht auf der Oberfläche des Linsenkörpers eine Zwischenschicht mit einem Brechungsindex der zwischen dem Brechungsindex der hydrophilen Schicht und dem Brechungsindex des Linsenkörpers liegt. Hierdurch wird im Gegensatz zu der aus US 2009/0018651 A1 bekannten Beschichtung aus einer oder mehreren Schichten zwischen dem Linsenkörpermaterial und der Beschichtung ohne zusätzliche Beschichtung ein reflektionsfreier Übergang zwischen Beschichtung und Linsenkörpermaterial erreicht. Unterstützt wird die Bildung dieses interpenetrierenden Polymer-Netzwerks durch die beschriebene Aktivierung der Oberfläche und die Folgebeschichtung.

Eine weitere Verbesserung des kontinuierlichen Übergangs der Brechungsindizes zwischen dem Augenkammerwasser und dem hydrophoben Linsenkörper der Intraokularlinse lässt sich noch dadurch erreichen, dass das Material der hydrophilen Schicht vom Kammerwasser angequollen wird. Im angequollenen Oberflächenbereich der hydrophilen Schicht liegt ein Brechungsindex vor, der zwischen dem Brechungsindex des Kammerwassers und dem Brechungsindex der hydrophilen Schicht liegt. Somit wird ein reflektionsfreier Übergang zwischen dem Kammerwasser und der hydrophilen Schicht auf dem Linsenkörper erreicht.

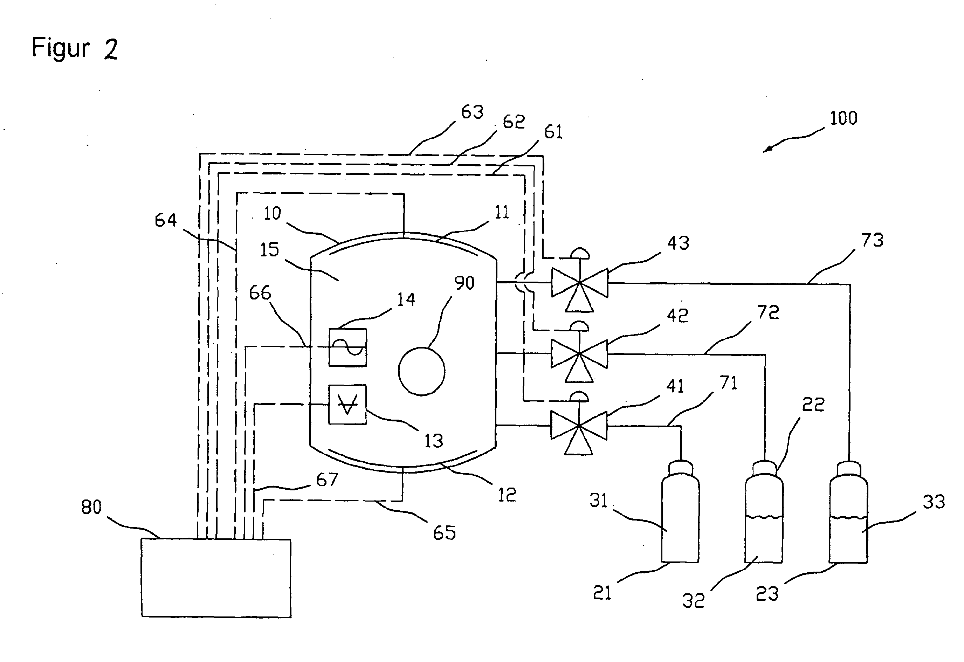

Das in Figur 2 dargestellte Schema veranschaulicht die wesentlichen Komponenten einer Vorrichtung 100 zur Beschichtung polymerer Werkstücke 90 mit einem deren Oberfläche hydrophil ausbildenden Material. Bei den Werkstücken handelt es sich um Intraokularlinsen, IOLs, und hierbei vorzugsweise um solche aus einem Silicon oder einem Siliconhydrogel.

Die Vorrichtung 100 umfasst einen evakuierbaren Rezipienten 10 mit einer Einrichtung zum Erzeugen eines Hochfrequenzplasmas im Innenraum 15 des Rezipienten 10. Die Einrichtung zum Erzeugen eines Hochfrequenzplasmas ist im Schema der Figur 2 mittels zweier Elektroden 11 und 12 symbolisiert, jedoch nicht auf die Verwendung von Elektroden beschränkt. In Figur 2 sind im Hinblick auf eine klare und übersichtliche Darstellung nur diejenigen Komponenten dargestellt, die als erforderlich für das Verständnis der Erfindung erachtet werden. Komponenten, wie z. B. Pumpen zum Evakuieren des Rezipienten 10, die für den Betrieb der Vorrichtung zwar unerlässlich sind, für das Verständnis der Erfindung jedoch ohne Bedeutung, sind im Rahmen dieser Offenbarung trotz fehlender Darstellung als vorhanden unterstellt. Dem Innenraum 15 des Rezipienten 10 sind zumindest eine Vakuum- bzw. Niederdruckmesseinrichtung 13 und eine Beschichtungsauftragsmesseinrichtung 14, z.B. ein Schwingquarz, zugeordnet.

Die Beschichtungsvorrichtung 100 weist ferner eine Inertgasquelle 21 und eine oder mehrere Beschichtungsmaterialquellen 22 und 23 auf. Jede der Quellen bzw. Quellbehälter 21, 22 und 23 ist jeweils über eine der Leitungen 71, 72 und 73 so mit dem Rezipienten 10 verbunden, dass in den Quellen vorrätig gehaltene gasförmige bzw. in die Dampfphase überführte Stoffe in den Innenraum 15 des Rezipienten 10 geleitet werden können. In den Leitungen 71, 72 und 73 angeordnete Steuerventile 41, 42 und 43 ermöglichen eine Regelung des jeweiligen Gas- bzw. Dampfflusses in den Rezipienten 10. In dem dargestellten Ausführungsbeispiel können die Steuerventile alternativ auch zum Entlüften der Quellbehälter 21, 22 und 23 verwendet werden. Bei anderen Ausführungsformen werden eigene Ventile oder separate Leitungen verwendet.

Die Vorrichtung 100 weist ferner eine Steuerung 80 auf, die z. B. mittels von Steuerleitungen 61, 62, 63, 64, 65 und Signalleitungen 66 und 67 zur gegebenenfalls geregelten Steuerung von Beschichtungsprozessen ausgebildet ist. Je nach Ausführungsform kann die Steuerung zur Ausführung vollautomatischer oder teilautomatischer Beschichtungsprozesse oder zur wahlweise voll- bzw. teilautomatischen Beschichtungssteuerung ausgebildet sein. In dieser Schrift wird übrigens nicht zwischen den Begriffen Steuern und Regeln unterschieden, d. h. der Begriff "Steuern" kann ebenso eine Rückführung einer Regelgröße bzw. deren Messwerts umfassen, wie sich der Begriff "Regeln" auf eine einfache Steuerkette beziehen kann. Eine geregelte (Teil-)Steuerung der Vorrichtung 100 kann z. B. unter Verwendung der Ausgangssignale von dem Innenraum 15 zugeordneten Sensoreinrichtungen realisiert sein. Beispielsweise können die Ventile 41, 42 und 43 unter Verwendung der Vakuum- bzw. Niederdruckmesseinrichtung 13 so gesteuert werden, dass im Innenraum 15 des Rezipienten 10 ein vorgegebener konstanter Gas- bzw. Dampfdruck mit ebenfalls vorgegebenen Partialdrücken aufrechterhalten wird. Ferner kann die Steuereinrichtung 80 dazu ausgebildet sein, das Aufwachsen einer Beschichtung mithilfe der Beschichtungsauftragsmesseinrichtung 14 zu überwachen und bei Erreichen der gewünschten Beschichtungsstärke zu beenden. Außerdem ist die Steuerung 80 in der Regel zur prozessablaufabhängigen Steuerung der Hochfrequenzeinrichtung 11 und 12 ausgebildet.

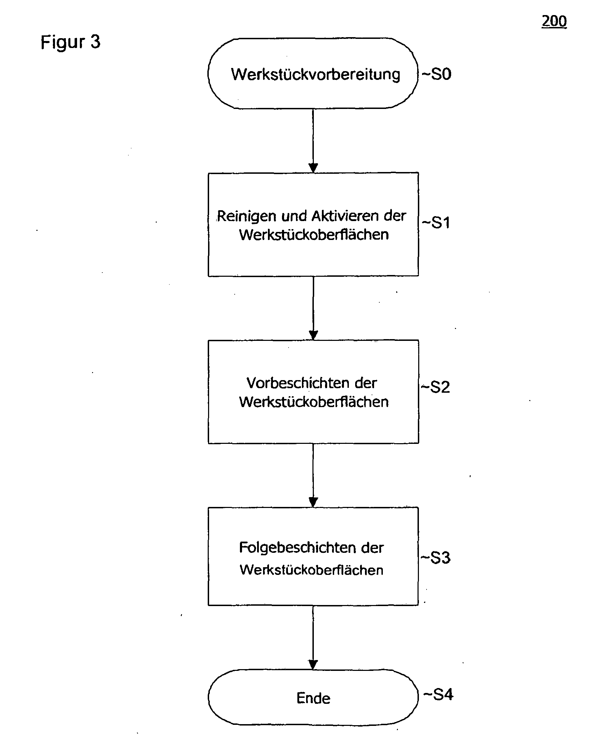

Das Flussdiagramm 200 der Figur 3 veranschaulicht die wesentlichen Schritte eines Verfahrens zum Hydrophilieren von Linsenoberflächen durch Beschichten mit Polyacrylsäure. Vorzugsweise bilden polymere Biomaterialien die zur Herstellung der Linsen 90 bzw. deren Oberflächenbereichen verwendeten Werkstoffe, wobei unter dem Begriff "Biomaterial" alle Werkstoffe verstanden werden, die zum Kontakt mit biologischem Gewebe oder Körperflüssigkeiten vorgesehen sind.

Nach der Vorbereitung der Werkstücke 90 in Schritt S0, die gegebenenfalls ein Reinigen der Werkstücke und deren Anordnen im Rezipienten 10 sowie ein anschließendes Evakuieren des Rezipienten umfasst, werden die Werkstückoberflächen zunächst in Schritt S1 für eine nachfolgende Beschichtung vorbereitet.

Hierzu wird der mit dem oder den Werkstücken beladene Rezipient 10 zunächst mithilfe von (in den Figuren nicht gezeigten) Pumpen evakuiert, vorzugsweise auf einen Druck von maximal 10⁻⁴ mbar (10 mPa). Nach Erreichen des gewünschten Vakuumdrucks wird das Rezipienteninnere 15 bei fortdauerndem Pumpen mit einem Inertgas, vorzugsweise Argon, geflutet, wobei der Inertgaszufluss so auf die Pumpleistung abgestimmt wird, dass sich im Innenraum 15 des Rezipienten 10 ein konstanter Gasdruck ausbildet. Das Inertgas 31 wird dem Rezipienten aus einer Inertgasquelle 21 zugeführt. In Ausführungsformen beträgt der so eingestellte Argongasdruck in etwa 25 mTorr (ca. 3,33 Pa). Nach Erreichen eines stabilen Inertgasdrucks im Rezipienteninneren 15 wird der Plasmagenerator, beispielsweise ein Hochspannungsgenerator, eingeschaltet, wodurch ein die Werkstücke 90 umgebendes Inertgasplasma geschaffen wird. Das Plasma reinigt die Linsenoberflächen durch Entfernen von daran adsorbierten Stoffen und resultiert ferner in einer Aktivierung der Linsenoberflächen durch Bildung von Ionen und freien Radikalen, die den nachfolgenden Aufpolymerisierungsprozess begünstigen.

Die Reinigungs- und Aktivierungswirkung kann in diesem ersten Schritt S1 über die Frequenz des Gasplasmas, die in das Plasma eingespeiste Leistung, die Einwirkzeit des Plasmas und die Art des für das Plasma verwendeten Inertgases wie allgemein bekannt beeinflusst werden. Die für den jeweiligen Anwendungsfall geeigneten Einstellungen können in üblicher Weise vom Fachmann ermittelt werden. Als Inertgas wird bei dem hier vorgestellten Verfahren Argon bevorzugt, da es eine Aktivierung der Werkstoffoberflächen ohne Erzeugen neuer unerwünschter Verbindungen ermöglicht. Selbstverständlich können stattdessen auch andere Inertgase verwendet werden, wie Stickstoff, wenn sie zu vergleichbaren Resultaten führen. Bei einer beispielhaften Ausführungsform des Verfahrens beträgt die Einwirkzeit des Argonplasmas in etwa eine Minute. Nach Ablauf der Einwirkzeit wird der Plasmagenerator ausgeschaltet und das Verfahren mit dem ersten Beschichtungsschritt S2 fortgeführt.

Abweichend vom bisher Dargestellten kann das zur Werkstückvorbereitung verwendete Plasma statt auf Basis reinen Argons auch auf Basis eines Gemisches von Inertgas und einer im nachfolgenden Vorbeschichtungsprozess verwendeten Reaktivkomponente erzeugt werden. Der Partialdruck der Reaktivkomponente sollte im Gasgemisch jedoch weniger als ein Zehntel des Partialdrucks des Inertgases betragen.

Beim Übergang von Schritt S1 zu Schritt S2 des Verfahrens wird der Inertgaszufluß ins Rezipienteninnere vorzugsweise aufrechterhalten und gegebenenfalls so geändert, dass er einen zur Durchführung von Schritt S2 geeigneten Wert aufweist. Zur Herstellung des Gasgemisches wird dem Inertgas im Rezipienten 10 ein aus biokompatiblen, polymerisierbaren carboxygruppenhaltigen Monomeren in der Dampfphase gebildetes erstes Schichtmaterialgas zugemischt. Bei den carboxygruppenhaltigen Monomeren handelt es sich vorzugsweise um Acrylsäure oder einen Acrylsäurevorläufer, wie z. B. (Meth)Acrylsäureanhydrid. Der Partialdruck P_{eSG} des ersten Schichtmaterialgases beträgt bei Ausführungsformen vorzugsweise mindestens ein Viertel und maximal das Doppelte des Partialdrucks p_{IG} des Inertgases. Besonders bevorzugt ist das Partialdruckverhältnis p_{eSG}=p_{IG} aus einem Bereich von 1:1 bis 1:0,5 ausgewählt. Beispielsweise beträgt der Partialdruck von Argon bei Ausführungsformen des Verfahrens 30 mTorr (ca. 400 mPa) bei einem Gesamtdruck des Gasgemisches von 45 mTorr (ca. 600 mPa), woraus sich ein Wert von 2:1 bezüglich des Verhältnisses von Argonpartialdruck p_{Ar} zu erstem Schichtmaterialgaspartialdruck (Reaktivkomponentenpartialdruck) P_{eSG} ergibt.

Als Reaktivkomponente zur Ausbildung des ersten Schichtmaterialgases wird bevorzugt (Meth)Acrylsäureanhydrid verwendet, das in einem der in Figur 1 veranschaulichten Behälter 22 oder 23 in die Dampfphase überführt und über die Leitung 72 oder 73 in den Innenraum 15 des Rezipienten 10 geleitet wird. Der Partialdruck des Schichtmaterialgases wird über dessen Zufluss eingestellt, der mithilfe der Ventile 42 oder 43 gesteuert wird. Statt (Meth)Acrylsäureanhydrid kann natürlich auch (Meth)Acrylsäure verwendet werden. (Meth)Acrylsäure bzw. (Meth)Acrylsäureanhydrid werden in den Quellbehältern 22 oder 23 in flüssiger Form, beispielsweise in einer Menge von 150 mL, bereitgestellt. Um ein Polymerisieren der Acrylsäure bzw. deren Vorläufermaterialien zu verhindern oder zu verzögern, können diese mit Cu(I)-chlorid versetzt werden. Ferner werden die Reaktivkomponentenbehälter 22 bzw. 23 nach dem Befüllen solange entlüftet, bis keine Blasen mehr in der Reaktivkomponentenflüssigkeit aufsteigen. Der Dampfdruck der Reaktivkomponenten ist bei den üblichen Raumtemperaturen von 22 bis 25°C zur Bildung des ersten Schichtmaterialgases in der Regel ausreichend.

Nach Einstellen des gewünschten Gasgemisches und Gasgemischdrucks wird der eigentliche Vorbeschichtungsvorgang durch Starten des Plasmagenerators eingeleitet, wodurch im Plasma erzeugte angeregte Acrylsäuremonomere sich an der aktivierten Werkstückoberfläche anlagern und im weiteren Verlauf dort eine Polyacrylsäureschicht ausbilden. Diese plasmaunterstützte Vorbeschichtungsphase wird aufrecht-erhalten, bis eine vorgegebene Schichtdicke erreicht ist. Das Aufwachsen der Beschichtung wird dabei fortlaufend mithilfe der Beschichtungs-auftragsmesseinrichtung 14 überwacht. Prinzipiell können Beschichtungen mit Dicken von bis zu 30.000 nm, respektive 30 µm abgeschieden werden, wobei ein jeweiliger Beschichtungsprozess beendet wird, sobald die Beschichtungsauftragsmesseinrichtung 14 das Erreichen der gewünschten Beschichtungsdicke innerhalb einer vorgegebenen Toleranz von z. B. 0,5 bis 4 Å anzeigt. Zum Hydrophilieren von IOLs haben sich Vorbeschichtungen mit Dicken aus einem Bereich von etwa 5 bis 40 A als geeignet erwiesen. Je nach der zu erzielenden Beschichtungsstärke kann die Vorbeschichtungsphase unter 10 Minuten, unter 3 Minuten oder unter 1 Minute dauern. Die Gaszufuhren werden während der Plasmabeschichtung vorzugsweise nicht verändert. Der Vorbeschichtungsprozess wird durch Abschalten des Plasmagenerators beendet.

Dem beschriebenen Vorbeschichtungsschritt S2 schließt sich der Folgebeschichtungsschritt S3 an, bei dem nach dem Ausschalten des Plasmagenerators zunächst der Inertgaszufluss unterbrochen und die vorbeschichtete Werkstückoberfläche dem möglichst vollen Dampfdruck einer von wasserfreier Acrylsäure gebildeten Reaktivkomponente ausgesetzt wird. Der Dampfdruck der Reaktivkomponente sollte 5 Torr (ca. 667 Pa) nicht unterschreiten. Leichtes Kühlen oder Erwärmen der Reaktivkomponente im Quellbehälter 22 oder 23 kann dabei zur Druckeinstellung zweckmäßig sein. Das Einbringen der Reaktivkomponente in den Rezipienten 10 bei vollem Dampfdruck stellt Reaktivgas in großen Mengen bereit, das mit den an der vorbeschichteten Oberfläche vorhandenen reaktiven Zentren reagiert und eine vergleichsweise dicke Polyacrylsäureschicht (PAA-Schicht) aufbaut, die kristallin sein kann.

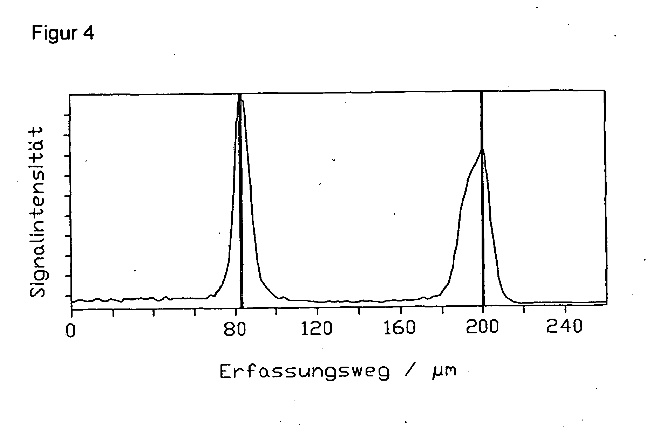

In Figur 4 ist ein Messdiagramm dargestellt, dem entnommen werden kann, dass eine wie oben beschrieben hergestellte PAA-Schicht eine Dicke von etwa 10 µm hat. Für die Messung wurde die hydrophile PAA-Schicht mit Rhodamin 6G als Fluoreszenzfarbstoff eingefärbt und die Fluoreszenz mittels konfokaler Mikroskopie dickenaufgelöst vermessen. Die hydrophile Schicht erstreckt sich, wie an dem rechten Bereich des Fluoreszenzsignalverlaufs deutlich ist, erkennbar in die Tiefe des Werkstücks. Die in Figur 4 vermessene (Meniskus-) Linse hat an der Messstelle eine Dicke von 117,5 µm. Die Auflösung der Messung beträgt 0,6 µm. Aus den erhaltenen Daten kann von einer Beschichtungsstärke auf den Oberflächen von ca. 10±0,6 µm (Bereich zwischen den senkrechten Strichen) und einer Eindringtiefe pro Seite von ca. 15 bis 20±0,6 µm ausgegangen werden. Das beschriebene Verfahren eignet sich daher besonders vorteilhaft zur Anwendung an Silicon-IOLs, bei denen Hydrophilie der Oberfläche, Dauerhaftigkeit der Beschichtung sowie deren optische Eigenschaften gleichermaßen bedeutend sind.

Nach Beendigung des Verfahrens in Schritt S4 können die beschichteten Werkstücke 90 aus dem Rezipienten entnommen und gegebenenfalls einer Qualitätskontrolle, Reinigung und Trocknung unterzogen werden.

Vor dem Beschichten wird der Linsenkörper, der hydrophob ist, mit zwei einander gegenüberliegenden konvexen Linsenflächen geformt, welche in Kontakt mit dem Glaskörper bzw. Kammerwasser die erforderliche Brechkraft bereitstellen. Die Beschichtung wird auf die fertiggeformte Linsenoberfläche nach Entnahme aus dem Formwerkzeug durch kombinierte PECVD (Vorbeschichtung) und CVD (Folgebeschichtung) aufgebracht, wobei PECVD für Plasma-Enhanced CVD steht und CVD für Chemical Vapor Deposition (also ohne Plasmaeinwirkung).

Das beschriebene Verfahren ermöglicht eine dauerhafte Hydrophilierung der Oberflächen von Intraokularlinsen aus Silikon, die eine ausgezeichnete Benetzung mit Wasser und damit eine hohe Bioverträglichkeit möglich machen.

Der Fachmann wird erkennen, dass zahlreiche Modifikationen und Abwandlungen der oben beschriebenen Beispiele möglich sind, ohne den Schutzbereich der beigefügten Ansprüche zu verlassen.

## Patentansprüche

1. Intraokularlinse mit einem hydrophoben Linsenkörper (1) aus Silikon, an dessen Oberfläche eine hydrophile Schicht (2) aus Polyacrylsäure, PAA, vorgesehen ist, **dadurch gekennzeichnet, dass**
der Benetzungswinkel von Wasser an der beschichteten Linsenoberfläche unter 10 Grad beträgt.

2. Intraokularlinse nach Anspruch 1, wobei in einer Oberflächenschicht des hydrophoben Linsenkörpers (1) Moleküle der hydrophilen Schicht eingelagert sind.

3. Intraokularlinse nach Anspruch 2, wobei die eingelagerten Moleküle der hydrophilen Schicht mit den Molekülen des Linsenkörperpolymers vernetzt sind.

4. Intraokularlinse nach Anspruch 2 oder 3, wobei die Oberflächenschicht des Linsenkörpers, in welchem die Moleküle der hydrophilen Schicht eingelagert sind, einen Brechungsindex zwischen den Brechungsindizes der hydrophilen Schicht (2) und des Linsenkörpers (1) aufweist.

5. Intraokularlinse nach einem der Ansprüche 1 bis 4, wobei die hydrophile Schicht (2) durch Wasser quellbar ist.

6. Intraokularlinse nach einem der Ansprüche 1 bis 5, wobei die Schicht (2) eine PECVD/CVD-Schicht ist.

7. Intraokularlinse nach einem der Ansprüche 1 bis 6, wobei der Benetzungswinkel von Wasser an der beschichteten Linsenoberfläche einen Wert aus dem Bereich von 2 bis unter 10 Grad oder aus dem Bereich unter 2 Grad aufweist.

8. Intraokularlinse nach einem der Ansprüche 1 bis 7, wobei die PAA-Schicht eine durchschnittliche Dicke von 5-40 µm aufweist.

9. Intraokularlinse nach einem der Ansprüche 1 bis 8, wobei der Linsenkörper (1) falt- oder rollbar ist.

10. Verfahren zum Hydrophilieren der Oberflächen einer Intraokularlinse, wobei das Verfahren Schritte umfasst zum
(a) Vorbehandeln der Linsenoberflächen zum Reinigen und Aktivieren der Linsenoberflächen in einem auf Grundlage eines Inertgases gebildeten Hochfrequenzgasplasma, und zum
(b) Vorbeschichten der so vorbehandelten Linsenoberflächen mit Polyacrylsäure unter Verwendung eines aus einer Gasmischung erzeugten Hochfrequenzgasplasmas, wobei sich die Gasmischung aus einem Inertgas und einem aus biokompatiblen, polymerisierbaren carboxygruppenhaltigen Monomeren gebildeten ersten Gas zusammensetzt, und ferner einen Schritt zum
(c) Folgebeschichten der so vorbeschichteten Linsenoberflächen,
wobei das Vorbeschichten gemäß Schritt (b) unter 10 Minuten dauert, und wobei
das Folgebeschichten gemäß Schritt (c) unter Verwendung eines im Wesentlichen Acrylsäuremonomere enthaltenden zweiten Gases geschieht.

11. Verfahren nach Anspruch 10, worin die das erste Gas bildenden Monomere ausgewählt sind unter (Meth)Acrylsäure und (Meth)Acrylsäureanhydrid.

12. Verfahren nach Anspruch 10 oder 11, worin das in Schritt (a) zur Ausbildung des Hochfrequenzplasmas .verwendete Gas das erste Gas in einer Menge enthält, die einem Partialdruck von weniger als einem Zehntel des Partialdrucks des Inertgases entspricht.

13. Verfahren nach einem der Ansprüche 10 bis 12, worin bei der in Schritt (b) verwendeten Gasmischung der Partialdruck des ersten Gases mindestens ein Viertel und maximal das Doppelte des Partialdrucks des Inertgases beträgt.

14. Verfahren nach einem der Ansprüche 10 bis 13, worin der Partialdruck des Inertgases im in Schritt (c) verwendeten zweiten Gas weniger als ein Zehntel des Partialdrucks des von Acrylsäuremonomeren gebildeten Gases beträgt.

15. Verfahren nach einem der Ansprüche 10 bis 14, *worin* die Intraokularlinse zumindest an ihrer Oberfläche überwiegend, ganz oder im Wesentlichen aus einem Silicon oder einem Siliconhydrogel gebildet ist.

16. Verfahren nach Anspruch 15, wobei die Intraokularlinse zumindest an ihrer Oberfläche überwiegend, ganz oder im Wesentlichen aus Poly(dimethylsiloxan) gebildet ist.

17. Verfahren nach Anspruch 15 oder 16, umfassend das Formen der Intraokularlinse, vor dem Beschichten, mit zwei einander gegenüberliegenden konvexen Linsenflächen zur Bereitstellung der erforderlichen Brechkraft des Auges.

## Claims

1. An intraocular lens with a hydrophobic lens body (1) made of silicone, on the surface of which a hydrophilic layer (2) of poly(acrylic acid), PAA, is provided,
**characterized in that**
the contact angle of water at the coated lens surface is less than 10 degrees.

2. The intraocular lens of claim 1, wherein in a surface layer of the hydrophobic lens body (1), molecules of the hydrophilic layer are embedded.

3. The intraocular lens of claim 2, wherein the embedded molecules are cross-linked to the molecules of the lens body polymer.

4. The intraocular lens of claim 2 or 3, wherein the surface layer of the lens body, in which the molecules of the hydrophilic layer are embedded, has a refractive index between the refractive indices of the hydrophilic layer (2) and of the lens body (1).

5. The intraocular lens of one of claims 1 to 4, wherein the hydrophilic layer (2) is water-swellable.

6. The intraocular lens of one of claims 1 to 5, present wherein the layer (2) is a PECVD/CVD double layer.

7. The intraocular lens of one of claims 1 to 6, wherein the water contact angle on the coated lens surface has a value in the range 2 to less than 10 degrees, or in the range below 2 degrees.

8. The intraocular lens of one of claims 1 to 7, wherein the PAA layer has an average thickness of 5-40 µm.

9. The intraocular lens of one of claims 1 to 8, wherein the lens body (1) is foldable or rollable.

10. A process of hydrophilizing the surfaces of an intraocular lens, the process comprising steps for
(a) pre-treating the lens surfaces for cleaning and activating the lens surfaces in a high frequency plasma formed on the basis of an inert gas, and for
(b) pre-coating the so-pretreated lens surfaces with polyacrylic acid using a high frequency plasma generated from a gas mixture, wherein the gas mixture is composed of an inert gas and a first gas formed of biocompatible, polymerizable, carboxy-group containing monomers, and further a step of
(c) follow-up coating the so-precoated lens surfaces,
wherein the pre-coating according to step (b) takes less than 10 minutes, and wherein
the follow-up coating according to step (c) occurs using a second gas substantially containing acrylic acid monomers.

11. The process of claim 10, wherein the monomers constituting the first gas are selected from (meth)acrylic acid and (meth)acrylic acid anhydride

12. The process of claim 10 or 11, wherein the gas used in step (a) for generating the high frequency plasma contains the first gas in an amount corresponding to a partial pressure of less than one tenth of the partial pressure of the inert gas.

13. The process of one of claims 10 to 12, wherein in the gas mixture used in step (b), the partial pressure of the first gas is at least one fourth of, and maximally twice the partial pressure of the inert gas.

14. The process of one of claims 10 to 13, wherein the partial pressure of the inert gas in the second gas used in step (c) is less than one tenth of the partial pressure of the gas formed of acrylic acid monomers.

15. The process of one of claims 10 to 14, wherein the intraocular lens is formed mainly, entirely or substantially from silicone or a silicone hydrogel at least in its surface region.

16. The process of claim 15, wherein the intraocular lens is formed mainly, entirely or substantially from poly(dimethylsiloxane) at least in its surface region.

17. The process of claim 15 or 16, comprising forming the intraocular lens, before coating it, with two mutually opposing convex lens surfaces for providing the required refractive power of the eye.

## Revendications

1. Lentille intraoculaire pourvue d'un corps de lentille (1) hydrophobe en silicone, à la surface duquel est prévue une couche (2) hydrophile en acide polyacrylique, PAA,
**caractérisée en ce que**
l'angle de mouillage de l'eau sur la surface de lentille revêtue est inférieur à 10 degrés.

2. Lentille intraoculaire selon la revendication 1, des molécules de la couche hydrophile étant stockées dans une couche superficielle du corps de lentille (1) hydrophobe.

3. Lentille intraoculaire selon la revendication 2, les molécules stockées de la couche hydrophile étant réticulées avec les molécules du polymère de corps de la lentille.

4. Lentille intraoculaire selon la revendication 2 ou 3, la couche superficielle du corps de la lentille, dans lequel les molécules de la couche hydrophile sont stockées, présentant un indice de réfraction compris entre les indices de réfraction de la couche (2) hydrophile et du corps de lentille (1).

5. Lentille intraoculaire selon l'une quelconque des revendications 1 à 4, la couche (2) hydrophile pouvant être gonflée par de l'eau.

6. Lentille intraoculaire selon l'une quelconque des revendications 1 à 5, la couche (2) étant une couche PECVD/CVD.

7. Lentille intraoculaire selon l'une quelconque des revendications 1 à 6, l'angle de mouillage de l'eau sur la surface de lentille revêtue présentant une valeur dans la plage allant de 2 à moins de 10 degrés ou dans la plage inférieure à 2 degrés.

8. Lentille intraoculaire selon l'une quelconque des revendications 1 à 7, la couche de PAA présentant une épaisseur moyenne de 5 à 40 µm.

9. Lentille intraoculaire selon l'une quelconque des revendications 1 à 8, le corps de lentille (1) pouvant être plié ou roulé.

10. Procédé d'hydrophilisation des surfaces d'une lentille intraoculaire, le procédé comprenant les étapes de
a) traitement préalable des surfaces de lentille pour nettoyer et activer les surfaces de lentille dans un plasma gazeux à haute fréquence formé sur la base d'un gaz inerte, et
b) revêtement préalable des surfaces de lentille préalablement traitées d'acide polyacrylique au moyen d'un plasma gazeux à haute fréquence produit à partir d'un mélange gazeux, le mélange gazeux étant composé d'un gaz inerte et d'un premier gaz constitué de monomères biocompatibles, polymérisables et contenant des groupes carboxy, ainsi qu'une étape de
c) revêtement subséquent des surfaces de lentille ainsi préalablement revêtues,
le revêtement préalable selon l'étape (b) durant moins de 10 minutes, et
le revêtement subséquent selon l'étape (c) étant mis en oeuvre au moyen d'un second gaz contenant sensiblement des monomères d'acide acrylique.

11. Procédé selon la revendication 10, au cours duquel les monomères formant le premier gaz sont sélectionnés parmi l'acide (meth)acrylique et l'anhydride d'acide meth(acrylique).

12. Procédé selon la revendication 10 ou 11, au cours duquel le gaz utilisé à l'étape (a) pour la formation du plasma à haute fréquence contient le premier gaz dans une quantité correspondant à une pression partielle inférieure à un dixième de la pression partielle du gaz inerte.

13. Procédé selon l'une quelconque des revendications 10 à 12, au cours duquel, pour le mélange gazeux utilisé à l'étape (b), la pression partielle du premier gaz atteint au moins un quart et au maximum le double de la pression partielle du gaz inerte.

14. Procédé selon l'une quelconque des revendications 10 à 13, au cours duquel la pression partielle du gaz inerte dans le second gaz utilisé à l'étape (c) est inférieure à un dixième de la pression partielle du gaz formé par les monomères d'acide acrylique.

15. Procédé selon l'une quelconque des revendications 10 à 14, au cours duquel la lentille intraoculaire est formée au moins sur sa surface, majoritairement, intégralement ou sensiblement, d'un silicone ou d'un silicone-hydrogel.

16. Procédé selon la revendication 15, la lentille intraoculaire étant constituée au moins sur sa surface, majoritairement, intégralement ou sensiblement, de poly(diméthylsiloxane).

17. Procédé selon la revendication 15 ou 16, comprenant la formation de la lentille intraoculaire, avant le revêtement, avec deux faces de lentille convexes se faisant face, ce qui permet d'obtenir la réfringence nécessaire de l'oeil.
